Europäisches Patentamt

European Patent Office

Office européen des brevets

(11) Veröffentlichungsnummer: **0 234 251 B1**

(12) **EUROPÄISCHE PATENTSCHRIFT**

(45) Veröffentlichungstag der Patentschrift: **01.04.92**

(21) Anmeldenummer: **87100641.7**

(22) Anmeldetag: **19.01.87**

(51) Int. Cl.5: **G01N 27/16**, G01N 27/14, G01N 33/00

(54) Verfahren und Vorrichtung zum Nachweis von brennbaren Gasen.

(30) Priorität: **21.01.86 GB 8601388**

(43) Veröffentlichungstag der Anmeldung:
**02.09.87 Patentblatt 87/36**

(45) Bekanntmachung des Hinweises auf die
Patenterteilung:
**01.04.92 Patentblatt 92/14**

(84) Benannte Vertragsstaaten:
**DE FR NL**

(56) Entgegenhaltungen:
**EP-A- 0 080 406**
**DE-A- 1 673 306**
**FR-A- 2 330 001**

**PATENT ABSTRACTS OF JAPAN, Band 6, nr.
53 (P-109)(931), 8 April 1982& JP A 56168543**

**SOVIET INVENTIONS ILLUSTRATED, Sektion
P1O, Woche 8621, 6 Juni 1986, Derwent Publications Ltd., London, GB, Klasse 503, Nr.
86136264/21; & SU A 1187051**

(73) Patentinhaber: **Drägerwerk Aktiengesellschaft
Moislinger Allee 53-55
W-2400 Lübeck 1(DE)**

(72) Erfinder: **Schuck, Hansjochen
Krogdiek 13
W-2406 Stockelsdorf(DE)**
Erfinder: **Iredale, Peter Julian Dr.
17 Woodkirk Close
Netherfield Park Seghill NE23 Tz(GB)**
Erfinder: **Johnson, Alan
11 Bedburn Avenue
Wear View Estate Sunderland(GB)**

## Beschreibung

Die Erfindung betrifft ein Verfahren zum Nachweis des Anteils von brennbaren Gasen in einem Luftgemisch mit Hilfe einer mit einer Anzeigeeinheit ausgestatteten Meßeinrichtung, die mittels eines einzigen, katalytisch aktiven Sensorelements sowohl nach dem Wärmetönungs- als auch nach dem Wärmeleitungsverfahren arbeitet und eine Anzeige nach dem Wärmetönungsverfahren nur unterhalb eines vorgebbaren Referenzwertes für das Meßsignal abgibt. Außerdem wird eine Meßeinrichtung zur Durchführung des Verfahrens angegeben.

Zum Nachweis und zur Messung von brennbaren Gasen werden bevorzugt Gassensoren eingesetzt, welche einen Katalysator enthalten und auf eine bestimmte Temperatur (beispielsweise 500°C) erhitzt werden, wodurch die brennbaren Gase unter Verbrauch eines Teiles des in dem Meßgas vorhandenen Sauerstoffs an der Sensoroberfläche katalytisch verbrennen und die Sensortemperatur erhöhen. Die bei der Verbrennungsreaktion auftretende Wärmetönung wird vermittels der Sensortemperaturerhöhung als Meßsignal für die Konzentration des brennbaren Gases in dem zu untersuchenden Luftgemisch ausgewertet und angezeigt. Als Meßeinrichtung wird dazu üblicherweise ein aktiver (Detektor) und ein passiver (Kompensator) Sensor in einem Brückenhalbzweig angeordnet. Diese Brücke wird entweder mit Konstantstrom oder Konstantspannung gespeist. Eine solche Meßeinrichtung arbeitet zufriedenstellend bis zu Konzentrationen von 100% UEG (Untere Explosionsgrenze) des brennbaren Gases in Luft. Diese Grenze beträgt für Methan beispielweise 5 Vol.-%. Oberhalb dieses Wertes führen in zunehmendem Maße Temperaturerhöhung des Detektors, Rest-Aktivität des Kompensators sowie Sauerstoffverarmung und thermische Leitfähigkeitsveränderung des Gasgemisches zu einem nichtlinearen Meßsignal bezüglich der Gaskonzentration. Bei Gaskonzentrationen in einem Bereich oberhalb von 100 % UEG ist das Ausgangssignal der Brücke nicht mehr eindeutig, d. h. man findet pro Wert der Ausgangsspannung zwei Gaskonzentrationen. Um eindeutige Messungen von Konzentrationen über 100 % UEG zu erreichen, wird beispielsweise die vorhandene Meßbrücke um einen weiteren Zweig ergänzt. Dieser mißt dann ebenfalls in einer Brückenanordnung bei einer reduzierten Arbeitstemperatur von beispielsweise 200 °C nur die Änderung der thermischen Leitfähigkeit des den Sensor umgebenden Gasgemisches. Bei Erreichen eines bestimmten Schwellwertes für die thermische Leitfähigkeit, welcher im Bereich von 100 % UEG liegen kann, wird über ein geeignetes Schaltelement das im Nullzweig der Wärmetönungsmeßbrücke angeschlossene Anzeigeelement automatisch auf Vollausschlag umgeschaltet. Dadurch wird angezeigt, daß die Gasprobe eine Konzentration an brennbaren Gasen von über 100 % UEG enthält.

Eine derartige Meßeinrichtung und das Verfahren zur Umschaltung zwischen Wärmeleitfähigkeits- und Wärmetönungsmessung ist in der DE-OS 16 73 306 beschrieben.

Bei der bekannten Meßeinrichtung wird beispielsweise zur Methanmessung in Luft ein erster Sensor in der Wärmetönungsmeßbrücke auf einer Betriebstemperatur von etwa 500 °C und ein weiterer Sensor in der Wärmeleitungsmeßbrücke auf einer Betriebstemperatur von beispielsweise 200 °C gehalten. Im Meßbereich bis zu 100 % UEG liefert der Sensor in der Wärmetönungsmeßbrücke ein eindeutiges Signal. Oberhalb der Grenze von 100 % UEG wird zwar das Meßgerät im Nullzweig der Wärmetönungsmeßbrücke auf Vollausschlag geschaltet, die Strom- bzw. Spannungsversorgung des Wärmetönungssensors bleibt jedoch erhalten. Bei weiterer Erhöhung des Methangehaltes in dem Luftgemisch über 100 % UEG hinaus erhöht sich die Verbrennungswärme an der Oberfläche des katalytischen Wärmetönungssensors derart, daß eine thermische Zerstörung der Katalysatorschicht, mindestens jedoch eine erhebliche Einschränkung der Brauchbarkeit des Sensors für weitere Messungen die Folge sein kann. Hier hilft dann auch kein Abschalten des Heizstromes mehr, um die Betriebstemperatur des Sensors zu senken, da die katalytische Verbrennung bei solch hohen Konzentrationen von selbst weiterläuft und so lange anhält, bis entweder die Gaskonzentration absinkt oder der Katalysator vergiftet bzw. der Sensor zerstört ist.

Nach dem Umschalten des im Nullzweig der Wärmetönungsmeßbrücke befindlichen Anzeigegerätes auf Vollausschlag ist eine Messung der Konzentration des tatsächlich im Luftgemisch vorhandenen nachzuweisenden Schadstoffes nicht mehr möglich.

Bei der bekannten Meßeinrichtung müssen zwei Meßbrücken zur gleichen Zeit kontinuierlich mit elektrischer Energie versorgt werden, insbesondere müssen beide in den Meßbrücken befindliche Sensoren auf 200 °C bzw. 500 °C angewärmt bleiben, um eine ständige Meßbereitschaft zu gewährleisten. Dies bedeutet einen erhöhten Energieverbrauch, da beide Sensoren gleichzeitig auf ihrer jeweiligen Betriebstemperatur gehalten werden müssen, obwohl nur einer von beiden für die tatsächliche Messung bzw. für die Bestimmung des Zeitpunktes für die Umschaltung des Meßgerätes auf Vollauschlag erforderlich wäre. Dieser erhöhte Energiebedarf verlangt bei entsprechender Einsatzdauer eines Meßgerätes eine darauf abgestimmte Energieversorgung, die beispielsweise bei einem tragbaren Meßgerät das Mitführen von geräumigen und schweren, und damit auch hinderlichen, Batterien notwendig macht.

Aus der JP-A-56 168543 wird eine Meßanordnung zur Messung brennbarer Gase mit einem einzigen Sensorelement beschrieben, das einen besonderen spezifischen Katalysatorzusatz aus $TiO_2 ZnO_2$ u.ä. enthält. Der Sensor wird auf eine einzige Betriebstemperatur, nämlich 350 - 400°C gehalten, bei der unter Anwesenheit von z.B. Propan, Butan eine katalytische Verbrennung stattfindet, und die Wärmetönung eine Temperaturerhöhung und damit eine Widerstandserhöhung in einer Meßbrücke nach sich zieht.

Dank des spezifischen Katalysatorzusatzes tritt bei Anwesenheit eines anderen Gases, z.B. Methan, an der Sensoroberfläche keine katalytische Verbrennung auf, sondern die Wärmeleitfähigkeit von Methan, welche größer ist als die der Luft, kühlt den Sensor ab und es findet eine Widerstandserniedrigung in der Messbrücke statt, was sich in einem Wärmeleitfähigkeitssignal äußert, dessen Polarität entgegen demjenigen der Wärmetönung verläuft.

Eine weitere bekannte Meßanordnung zur Messung brennbarer Gase aus der SU-A-1187051 sieht vor, daß bei einer Wärmetönungsmessung zur Vermeidung einer Selbstzerstörung des Katalysatorelements infolge Eigenkatalyse bei zu hohen Gaskonzentrationen durch Umschaltung der Betriebsart vom D.C.-Mode auf Constant-Current-Mode der Betriebsstrom auf ein bestimmtes sicheres Niveau begrenzt wird.

Der Sensor bleibt dabei auf einer hohen, jedoch durch den Betriebsstrom begrenzten Betriebstemperatur, um weiterhin - ohne durch Eigenkatalyse zerstört zu werden - in der Betriebsart der Wärmetönungsmessung arbeiten zu können. Erst bei kleiner werdenden Meßgaskonzentrationen steigt der Stromwert wieder über den vorher festgehaltenen Grenzstrom an.

Der vorliegenden Erfindung liegt somit die Aufgabe zugrunde, ein Verfahren zum Nachweis von brennbaren Gasen der bekannten Art so zu verbessern, daß eine eindeutige Messung des brennbaren Gases in Luft im gesamten Konzentrationsbereich von 0 bis 100 Vol.-% bei geringstmöglichem Energieverbrauch möglich ist. Dabei soll die Meßgenauigkeit im unteren Meßbereich bis etwa zur Unteren Explosionsgrenze besonders hoch sein. Ein Meßgerät, welches mit einem derartigen verbesserten Verfahren betrieben werden kann, soll ein kleineres Volumen besitzen, leichter und zum Zwecke der Wartung einfacher sein und aus weniger Teilen bestehen. Die Lebensdauer des Nachweiselementes soll erhöht werden.

Die Lösung der Aufgabe erfolgt durch ein Verfahren, welches durch die Verwendung eines einzigen, bei höheren Temperaturen katalytisch aktiven Sensorlementes gekennzeichnet ist, wobei der Sensor zu Beginn der Messung auf einer für die Wärmeleitfähigkeitsmessung ausreichenden ersten Temperatur $T_1$ konstant gehalten, und während der Messung das resultierende Meßsignal mit einem Referenzwert $R_1$ verglichen und wenn es den Referenzwert $R_1$ unterschreitet, die Temperatur des Sensors auf eine die Wärmetönungsmessung ermöglichende zweite Tempertur $T_2$ erhöht und auf dieser konstant gehalten wird und das vom Sensor abgegebene Meßsignal mit einem, einer höheren Gaskonzentration als $R_1$ entsprechenden Belastungsgrenzwert G verglichen und seine Temperatur auf die die Wärmeleitfähigkeitsmessung ermöglichende Temperatur $T_1$ abgesenkt und konstant gehalten wird, sobald das Meßsignal den Grenzwert G überschreitet.

Wird der Sensor in Betrieb genommen, erfolgt im ersten Verfahrensschritt eine Überprüfung, ob überhaupt brennbares Gas vorhanden ist. Dies kann dadurch erfolgen, daß der Sensor bei einer niedrigen, wenig elektrische Leistung verbrauchenden Betriebstemperatur $T_1$ zur Wärmeleitfähigkeitsmessung eingesetzt wird. Wird durch das resultierende Meßsignal angezeigt, daß brennbares Gas in Konzentrationen kleiner als dem Wert von $R_1$ entsprechend vorhanden ist, wird die Betriebsart des Sensors auf die Wärmetönungsmessung umgeschaltet und seine Betriebstemperatur auf $T_2$ erhöht. Die Wärmetönungsmessung kann genauer ausgeführt werden als die Wärmeleitfähigkeitsmessung, und die Konzentration des brennbaren Gases im Luftgemisch wird genauer angezeigt. Der Referenzwert $R_1$ kann somit als Schwellwert angesehen werden, welcher das Vorhandensein von brennbaren Gasen markiert und den Sensor schon frühzeitig, jedenfalls vor Erreichen der unteren Explosionsgrenze des Gasgemisches, bei solch geringen Konzentrationen an brennbarem Gas auf die genauere Wärmetönungsmessung umschaltet, die eine Schädigung des Sensors nicht hervorrufen können. Wenn sich der Sensor in der Betriebsart der Wärmetönungsmessung befindet, wird das von ihm abgegebene Meßsignal mit einem Belastungsgrenzwert G verglichen. Solange das Meßsignal den Grenzwert G unterschreitet, bleibt der Sensor in der Betriebsart der Wärmetönungsmessung. Wird z. B. bei steigender Gaskonzentration dieser Grenzwert G erreicht, wird der Sensor auf die Betriebsart der Wärmeleitfähigkeitsmessung und die dazu niedrigere Betriebstemperatur $T_1$ umgeschaltet. In dieser Betriebsart wird dann die Messung bei weiter ansteigenden Gaskonzentrationen durchgeführt, wobei die Meßwerte in eindeutiger Weise den entsprechenden Gaskonzentrationen zugeordnet werden können. Durch die Festlegung des Grenzwertes G wird erreicht, daß bei ansteigender Gaskonzentration die Umschaltung auf die niedrigere Temperatur $T_1$ schon erfolgt, bevor eine Selbsterhaltung der katalytischen Verbrennung auch ohne jede elektrische Energiezufuhr eintreten und den Sensor beschädigen oder zerstören könnte. Im genaueren Meßbetrieb der Wärmetönungsmessung wird der Sensor auf der dazu notwendigen Temperatur $T_2$ konstant gehalten, ohne daß ein unkontrolliertes Aufheizen beim Vorhandensein von brennbaren Gasen möglich ist. Das frühzeitige Umschalten in die Betriebsart der Wärmeleitfähigkeitsmessung schützt den Sensor bei erhöhten Konzentrationen von brennbarem Gas vor der Zerstörung seiner zur Wärmetönungsmessung notwendigen katalytischen Oberfläche. Dadurch wird die Lebensdauer des Sensors erhöht und der Leistungsverbrauch auf das zur Aufrechterhaltung der Betriebstemperatur notwendige Maß begrenzt.

Es ist zweckmäßig, die Lage der Referenzwerte $R_1$ und G in den Bereich zwischen l00 % UEG und l40 % UEG zu legen. Somit werden alle unterhalb dieses Bereiches liegenden Meßwerte nach dem Verfahren der Wärmetönungsmessung erfaßt.

In einem abgewandelten Verfahren kann vorgesehen sein, daß die Wärmetönungsmessung erst dann durchgeführt wird, wenn das Meßsignal innerhalb eines Fensters zwischen den Referenzwerten $R_1$ und $R_2$ liegt. Somit kann ein unterer Schwellwert festgelegt werden, unterhalb dessen die energiesparende Wärmeleitfähigkeitsmessung durchgeführt wird und erst bei Überschreiten des Referenzwertes $R_2$ der Sensor in die genauere, jedoch mehr Energie verbrauchende Wärmetönungsmessung umgeschaltet wird.

Dieses Verfahren ist insbesondere dann vorteilhaft, wenn über längere Zeiträume hinweg ein Auftreten von brennbaren Gasen in wesentlichen Konzentrationen, d. h. oberhalb dem Referenzwert $R_2$ entsprechend, nicht erwartet wird.

Der Referenzwert $R_1$ bzw. der Belastungsgrenzwert G können etwa bei l00 - l40 % der Unteren Explosionsgrenze (UEG), der zweite Referenzwert $R_2$ im Bereich zwischen 2 % und 5 % UEG liegen. Somit wird das Verfahren dazu eingesetzt, vorteilhafterweise den Konzentrationsbereich unterhalb der Unteren Explosionsgrenze bei wesentlichen Konzentrationen mit dem genauen Meßverfahren der Wärmetönungsmessung bei erhöhter Betriebstemperatur $T_2$ des Sensors zu überwachen und, sobald die Untere Explosionsgrenze erreicht wird, den Sensor nach dem Meßverfahren der Wärmeleitfähigkeitsmessung bei niedrigerer Betriebstemperatur $T_1$ des Sensors zu betreiben. Sobald die Konzentration des brennbaren Gases unter die Untere Explosionsgrenze sinkt, wird der Sensor automatisch auf das Meßverfahren der Wärmetönungsmessung zurückgeschaltet. Somit wird verhindert, daß der Sensor bei erhöhten Konzentrationen oberhalb der unteren Explosionsgrenze im Betriebsverfahren der Wärmetönungsmessung durch die katalytische Verbrennung beschädigt oder gar zerstört wird, da seine Betriebstemperatur frühzeitig auf die niedrigere Stufe der Wärmeleitfähigkeitsmessung umgeschaltet wurde. Deshalb ist er auch in der Lage, nach Abklingen der hohen Konzentration an brennbaren Gasen erneut nach der Betriebsart der Wärmetönungsmessung genaue Meßwerte zu liefern.

Es kann wünschenswert sein, im oberen Meßbereich lediglich einen Vollausschlag des Anzeigeinstrumentes auszulösen, um die vorhandene Gefahr zu kennzeichnen.

Darüber hinaus kann bei Überschreiten des zweiten Grenzwertes eine Alarmanlage angesteuert werden.

Eine zur Durchführung des Verfahrens geeignete Meßeinrichtung besteht aus einem einzigen Sensor, welcher mittels einer steuerbaren Stromquelle auf einer vorgegebenen Temperatur gehalten ist, welche ständig gemessen, überwacht und bezüglich der Umgebungstemperatur kompensiert wird. Ein Rechenwerk vergleicht das Sensorsignal mit vorgegebenen Referenzwerten $R_1$, $R_2$ bzw. dem Belastungsgrenzwert G und entscheidet je nach Größe des Meßsignals, ob der Sensor in der Betriebsart der Wärmetönungsmessung oder in der Betriebsart der Wärmeleitfähigkeitsmessung betrieben werden soll. Ein entsprechendes Signal wird an die Stromquelle abgegeben. Somit ist es möglich, nur mit einem einzigen Sensor beide Betriebsarten zur Messung der Konzentration eines brennbaren Gases mit einem für beide Betriebsarten brauchbaren Meßkreis durchzuführen.

Ein Ausführungsbeispiel der Erfindung wird anhand der Zeichnung dargestellt und im folgenden näher beschrieben.

Es zeigen:

Figur l ein Blockschaltbild der Meßeinrichtung zur Durchführung des Verfahrens,

Figur 2 ein Kennliniendiagramm der Wärmetönungs- und Wärmeleitfähigkeitsmessung,

Figur 3 den Bereich um den Ursprungspunkt aus Figur 2 in vergrößerter Darstellung.

In Figur l ist der für die Wärmeleitfähigkeits- und Wärmetönungsmessung geeignete Sensor l an eine steuerbare Stromquelle 2 und einen Arbeitswiderstand 3 angeschlossen. Über dem Sensor l wird mit Hilfe des Verstärkers 4 der Sensorwiderstand gemessen und in einem Rechenwerk 5, 6 als dessen Temperaturwert verarbeitet. Das Meßsignal des Sensors wird über eine Meßleitung 7 an den positiven Eingang eines Meßverstärkers 8 gegeben, dessen Ausgang über die Signalleitung 9 an eine Recheneinheit 5 angeschlossen ist. Die Recheneinheit 5 fragt über die Abfrageleitungen l0, ll ständig die

Referenzwerte $R_1$ und $R_2$ bzw. den Belastungsgrenzwert G in dem Vergleicher 6 ab. Je nach Höhe der Temperatur des Sensors I und je nach Lage des Meßwertes in bezug auf die Referenzwerte $R_1$ und $R_2$ bzw. G wird von dem Vergleicher 6 über eine Rückkopplung I2 die Stromquelle 2 derart angesteuert, daß sie entweder eine zur Konstanthaltung der Temperatur des Sensors I oder zur Umschaltung zwischen den Betriebstemperaturen der Wärmeleitfähigkeitsmessung und Wärmetönungsmessung notwendige Stromstärke an den Sensor I abgibt. Der Sollwert wird in Abhängigkeit von der Umgebungstemperatur über einen Temperaturfühler korrigiert. Der von der Recheneinheit 5 ermittelte Meßwert wird über eine Anzeigeeinheit I3 angezeigt. Bei Überschreiten des Meßsignals aus der Signalleitung 9 über den Belastungsgrenzwert G wird eine Alarmeinrichtung I4 angesteuert, um ein Überschreiten des Grenzwertes akustisch oder optisch anzuzeigen.

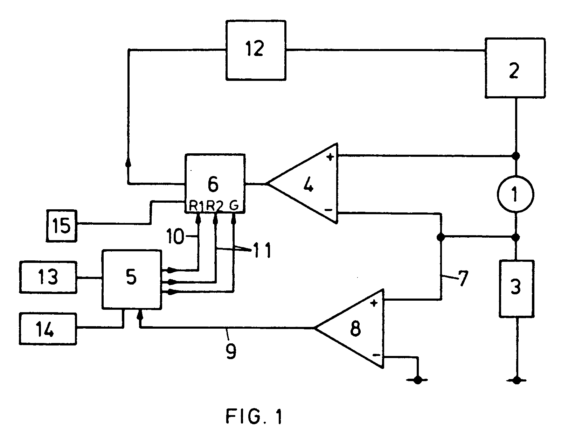

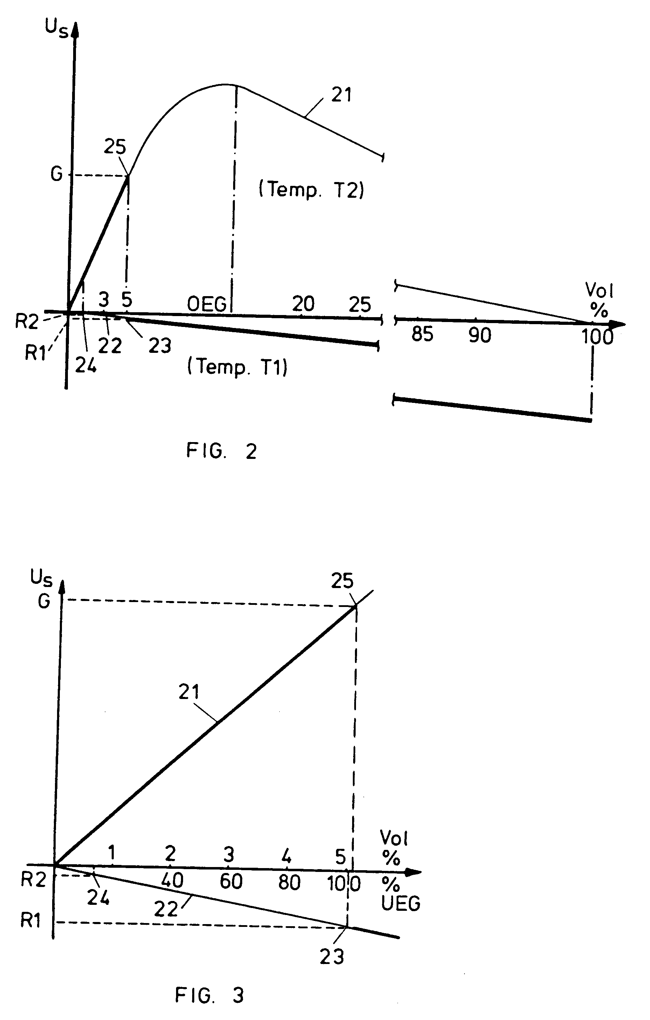

In Figur 2 und als vergrößerter Ausschnitt in Figur 3 sind zwei Meßkurven einer Methanmessung des Sensors I aufgezeichnet. Die erste Meßkurve 2I zeigt den Verlauf des Meßsignals $U_s$ in Abhängigkeit von der Konzentration des brennbaren Gases im Luftgemisch in Volumenprozent an. Sie gilt für die Betriebsart des Sensors I für die Wärmetönungsmessung. Ihre Schnittpunkte mit der Abszisse liegen im Ursprungspunkt und bei I00 Vol.-%. Dazwischen durchläuft sie ein Maximum. Die zweite Meßkurve 22 zeigt den linearen Signalverlauf des Meßsignals des Sensors I für die Betriebsart der Wärmeleitfähigkeitsmessung. Sie beginnt im Ursprung des Koordinatensystems und besitzt eine monotone negative Steigung. Auf der Ordinate sind die Referenzwerte $R_1$, $R_2$ und der Grenzwert G eingetragen. Ihre Schnittpunkte mit den entsprechenden Meßkurven liegen bei 23 und 24 bzw. 25.

Bei Inbetriebnahme der Meßeinrichtung wird der Sensor I von der Stromquelle 2 zunächst auf die für die Wärmeleitfähigkeitsmessung geeignete Betriebstemperatur $T_1$ eingestellt. Seine Meßwerte liegen auf der Meßkurve 22. Werden Meßwerte von der Recheneinheit 5 erfaßt, die unterhalb des Grenzwertes $R_1$ liegen, erkennt das Rechenwerk 5, 6, daß brennbares Gas in niedriger Konzentration vorliegt, und der Sensor wird auf eine die Betriebsart der Wärmetönungsmessung ermöglichende Betriebstemperatur $T_2$ gebracht. Alle nun erfolgenden Messungen werden aufgrund der Wärmetönungsmessung auf der Meßkurve 2I liegen. Solange das Meßsignal $U_s$ unterhalb des Grenzwertes G liegt, bleibt der Sensor I in der Betriebsart der Wärmetönungsmessung. Sobald jedoch das Meßsignal den Grenzwert G überschreitet, stellt das Rechenwerk 5, 6 fest, daß Konzentrationen von brennbarem Gas oberhalb beispielsweise 5 Vol.-% erreicht wurden, und veranlaßt nun die Stromquelle 2, die Betriebstemperatur des Sensors I auf die für die Betriebsart der Wärmeleitfähigkeitsmessung notwendige niedrigere Betriebstemperatur $T_1$ abzusenken. Bei weiterer Erhöhung der Konzentration des brennbaren Gases erfolgt nun die Messung entlang der Meßkurve 22 auf dem dick gekennzeichneten Teilbereich.

Sinkt die Konzentration des brennbaren Gases unterhalb 5 Vol.-% ab, wird der Sensor I wieder auf die Betriebsart der Wärmetönungsmessung umgeschaltet, und die weitere Messung erfolgt wieder entlang der dick ausgezogenen Teilstrecke der Meßkurve 2I. Auf diese Weise erfolgt je nach Höhe der Konzentration des brennbaren Gases ein abwechselndes Umschalten von einer Betriebsart zur anderen.

Soll die Meßeinrichtung in Abwandlung des Betriebsverfahrens erst bei Überschreiten eines weiteren Referenzwertes $R_2$ auf die Betriebsart der Wärmetönungsmessung umgeschaltet werden, überprüft der Vergleicher 6, ob die resultierenden Meßsignale innerhalb eines Fensters zwischen $R_1$ und $R_2$ liegen.

In Figur 2 wurde in nicht maßstäblicher Darstellung das Beispiel einer Methanmessung dargestellt, bei der die Konzentration von 5 Vol.-% der I00 % Unteren Explosionsgrenze entspricht. Zur Information ist, ebenfalls nicht maßstäblich, die Grenzlinie der Oberen Explosionsgrenze (OEG) dargestellt. In ganz analoger Weise erfolgt die Messung anderer brennbarer Gase.

**Patentansprüche**

1. Verfahren zum Nachweis des Anteils von brennbaren Gasen in einem Luftgemisch mit Hilfe einer mit einer Anzeigeeinheit ausgestatteten Meßeinrichtung die mittels eines einzigen, katalytisch aktiven Sensorelementes (1), sowohl nach dem Wärmetönungsverfahren als auch nach dem Warmeleitungsverfahren arbeitet und eine Anzeige nach dem Wärmetönungsverfahren nur unterhalb eines vorgebbaren Referenzwertes für das Meßsignal abgibt, gekennzeichnet durch die Verfahrensschritte, daß

a) der Sensor (1) zu Beginn der Messung auf einer für die Wärmeleitfähigkeitsmessung ausreichenden ersten Temperatur $T_1$ konstant gehalten, und während der Messung

b) das resultierende Meßsignal mit einem Referenzwert $R_1$ verglichen und

c) wenn es den Referenzwert $R_1$ unterschreitet, die Temperatur des Sensors (1) auf eine die Wärmetönungsmessung ermöglichende zweite Temperatur $T_2$ erhöht und auf dieser konstant gehalten wird, und

d) das vom Sensor (1) abgegebene Meßsignal mit einem, einer höheren Gaskonzentration als $R_1$ entsprechenden Belastungsgrenzwert G verglichen und seine Temperatur auf die die Wärmeleitfähigkeitsmessung ermöglichende Temperatur $T_1$ abgesenkt und konstant gehalten wird, sobald das Meßsignal den Grenzwert G überschreitet.

2. Verfahren nach Anspruch 1, **dadurch gekennzeichnet**, daß sowohl der Referenzwert $R_1$ als auch der Belastungsgrenzwert G etwa im Bereich von l00 % bis l40 % der unteren Explosionsgrenze (UEG) liegen.

3. Verfahren nach Anspruch 1, **dadurch gekennzeichnet**, daß das resultierende Meßsignal zusätzlich zu Verfahrensschritt b) mit einem zweiten Referenzwert $R_2$, dessen entsprechende Gaskonzentration unterhalb derjenigen des Referenzwertes $R_1$ liegt, verglichen wird und die Temperaturerhöhung des Sensors (l) nach Verfahrensschritt c) dann erfolgt, wenn das Meßsignal zusätzlich den Referenzwert $R_2$ überschreitet.

4. Verfahren nach Anspruch 3, **dadurch gekennzeichnet**, daß zur Messung von Methan der Referenzwert $R_1$ bzw. der Belastungsgrenzwert G etwa l00 - l40 % UEG des brennbaren Gases entsprechen und der zweite Referenzwert $R_2$ im Bereich zwischen 2 % und 5 % UEG liegt.

5. Verfahren nach einem der Ansprüche l oder 3, **dadurch gekennzeichnet**, daß der Sensor (l) im Schritt d) die Anzeigeeinheit (l3) der Meßeinrichtung auf Vollausschlag bringt.

6. Verfahren nach Anspruch 5, **dadurch gekennzeichnet**, daß eine Alarmeinrichtung (l4) angesteuert wird.

7. Meßeinrichtung zur Durchführung des Verfahrens nach einem der Ansprüche l oder 3, **dadurch gekennzeichnet**, daß ein einziger, katalytisch aktiver Sensor (l) an eine steuerbare Stromquelle (2) angeschlossen ist, dessen Ausgangssignal über einen Meßverstärker (8) an ein Rechenwerk (5, 6) geführt ist, das über eine Rückkopplungseinheit (l2) zur Abgabe eines Steuersignals derart an die Stromquelle (2) angeschlossen ist, daß diese aufgrund eines Vergleichs des über einen Verstärker (4) gemessenen Sensorwiderstandes mit den in einem Vergleicher (6) gespeicherten Referenzwerten $R_1$, $R_2$ bzw. Belastungsgrenzwert G die für die Aufrechterhaltung der Temperaturen $T_1$, $T_2$ notwendigen Stromstärken an den Sensor (l) abgibt.

## Claims

1. Method for detecting the content of combustible gases in an air mixture with the aid of a measuring device which is equipped with a display unit and which by means of a single catalytically active sensor element (1) operates both according to the heat of reaction method and according to the heat-conduction method and only supplies a display according to the heat of reaction method below a reference value for the measurement signal, which reference value can be preset, characterised by the method steps in which
    a) the sensor (1) at the beginning of the measurement is kept constant at a first temperature $T_1$ which suffices for the heat-conductivity measurement, and during the measurement
    b) the resultant measurement signal is compared with a reference value $R_1$, and
    c) if there is a falling below the reference value $R_1$, the temperature of the sensor (1) is increased to a second temperature $T_2$ rendering possible the heat of reaction measurement and is kept constant at this temperature, and
    d) the measurement signal supplied by the sensor (1) is compared with a load limiting value G which corresponds to a higher gas concentration than $R_1$ and its temperature is lowered to the temperature $T_1$ which renders possible the heat-conductivity measurement and is kept constant as soon as the measurement signal exceeds the limiting value G.

2. Method according to claim 1, characterised in that both the reference value $R_1$ and the load limiting value G lie substantially in the range of 100 % to 140 % of the lower explosive limit (UEG).

3. Method according to claim 1, characterised in that the resultant measurement signal, in addition to method step b), is compared with a second reference value $R_2$, the corresponding gas concentration of which lies below that of the reference value $R_1$, and the temperature increase of the sensor (1) according to method step c) occurs if the measurement signal in addition exceeds the reference value $R_2$.

4. Method according to claim 3, characterised in that for the purpose of measuring methane the

reference value $R_1$ or the load limiting value G substantially corresponds to 100 - 140 % of the lower explosive limit of the combustible gas and the second reference value $R_2$ lies in the range between 2 % and 5 % of the lower explosive limit.

5. Method according to one of the claims 1 or 3, characterised in that the sensor (1) in step d) brings the display unit (13) of the measuring device to full-scale deflection.

6. Method according to claim 5, characterised in that an alarm device (14) is activated.

7. Measuring device for carrying out the method according to one of the claims 1 or 3, characterised in that a single catalytically active sensor (1) is connected to a controllable current source (2), the output signal of which sensor is conducted, by way of a measuring amplifier (8), to an arithmetic unit (5, 6) which is connected, by way of a feedback unit (12) for the supply of a control signal, to the current source (2) in such a way that the latter, on the basis of a comparison of the sensor resistance, measured by way of an amplifier (4), with the reference values $R_1$, $R_2$, stored in a comparator (6), or load limiting value G respectively, supplies the necessary intensities of current for maintaining the temperatures $T_1$, $T_2$ to the sensor (1).

**Revendications**

1. Procédé destiné à déceler la proportion de gaz combustibles contenus dans un mélange d'air, à l'aide d'un dispositif de mesure équipé d'une unité d'affichage, qui au moyen d'un seul élément de capteur (1) actif catalytiquement, fonctionne suivant le principe de la chaleur de réaction ainsi que suivant le principe de la conduction de chaleur et ne fournit une indication pour le signal de mesure selon le procédé de chaleur de réaction qu'au-dessous d'une valeur de référence donnée pour le signal de mesure, caractérisé par les étapes de procédé suivantes:
    a) au début de la mesure, le capteur (1) est maintenu constant sur une première température $T_1$ suffisante pour la mesure de la conductibilité thermique et pendant la mesure
    b) le signal de mesure résultant est comparé à une valeur de référence $R_1$ et
    c) s'il est inférieur à la valeur de référence $R_1$, la température du capteur (1) est augmentée jusqu'à une deuxième température

$T_2$ permettant la mesure de chaleur de réaction et maintenu constant sur celle-ci et
    d) le signal de mesure délivré par le capteur (1) est comparé à une valeur limite de charge G correspondant à une concentration de gaz supérieure à $R_1$ et sa température est abaissée à la température $T_1$ permettant la mesure de la conductibilité thermique et maintenue constante, dès que le signal de mesure dépasse la valeur limite G.

2. Procédé selon la revendication 1, caractérisé en ce que la valeur de référence $R_1$ ainsi que la valeur limite de charge G se situent à peu près dans le domaine de 100 % à 140 % de la limite inférieure d'explosibilité (UEG).

3. Procédé selon la revendication 1, caractérisé en ce que le signal de mesure résultant est comparé, en plus de l'étape de procédé b), à une deuxième valeur de référence $R_2$ dont la concentration de gaz correspondante se situe au-dessous de celle de la valeur de référence $R_1$ et l'augmentation de la température du capteur (1) s'effectue ensuite selon l'étape de procédé c), lorsque le signal de mesure est supérieur en outre à la valeur de référence $R_2$.

4. Procédé selon la revendication 3, caractérisé en ce que pour mesurer du méthane, la valeur de référence $R_1$ ou la valeur limite de charge G correspond à peu près à entre 100 et 140 % de la limite inférieure d'explosibilité du gaz combustible et la deuxième valeur de référence $R_2$ se situe entre 2 % et 5 % de cette limite.

5. Procédé selon la revendication 1 ou 3, caractérisé en ce que dans l'étape d), le capteur (1) fait dévier l'aiguille de l'unité d'affichage (13) du dispositif de mesure sur sa valeur maximale.

6. Procédé selon la revendication 5, caractérisé en ce qu'un dispositif d'alarme (14) est commandé.

7. Dispositif de mesure pour la mise en oeuvre du procédé selon la revendication 1 ou 3, caractérisé en ce qu'un seul capteur (1) actif catalytiquement, est raccordé à une source de tension (2) commandable dont le signal de sortie est envoyé, par un amplificateur de mesure (8), à un calculateur (5, 6) qui est raccordé, par une unité de rétro-action (12), à la source de tension (2), pour délivrer un signal de commande, de telle sorte que sur la base d'une comparaison entre la résistance du capteur mesurée par l'intermédiaire d'un amplificateur (4) et les valeurs de référence $R_1$, $R_2$ ou la valeur limite de charge G, stockées dans un comparateur (6), la source de tension délivre au capteur (1) les intensités nécessaires au maintien des températures $T_1$, $T_2$.

FIG. 1

EP 0 234 251 B1

FIG. 2

FIG. 3